# DEMANDE DE BREVET EUROPEEN

(11) **EP 0 880 950 A1**
(43) Date de publication de la demande: **02.12.1998**
(21) Numéro de dépôt: 98401273.2
(22) Date de dépôt: 28.05.1998
(51) Int. Cl.: A61F 2/44

(54) **Cage intervertébrale cervicale**

(30) Priorité: 30.05.1997 FR 9706764
(71) Demandeur: BIOMAT, 91892 Saclay (FR); Cottin, Philippe, 78740 Saint Remy les Chevreuses (FR)
(72) Inventeur: Lahille, Michel, 91430 Vauhallan (FR); Cottin, Philippe, 78740 Saint remy les Chevreuse (FR)
(74) Mandataire: Cabinet Martinet & Lapoux

(57) **Abrégé**

La cage ne comprend qu'un corps monolithique (C) et une vis (5). Le corps à insérer entre deux corps vertébraux (CV1, CV2) comprend deux branches (1, 2) et une âme (3) reliant des extrémités postérieures (13, 23) des branches. La vis (5) est manoeuvrable depuis des extrémités antérieures (11, 21) des branches pour les écarter angulairement après insertion de la cage entre les corps vertébraux. La tige filetée (42) de la vis s'étend entre les branches (1, 2) et est vissée dans l'âme (3). L'âme comporte un bossage (32) dans lequel la tige de vis (53) est vissée et qui saille dans une double lumière pratiquée dans le corps de cage monolithique (C) entre les branches (1, 2) et l'âme. La cage présente ainsi des branches ayant une flexion relativement élevée comparativement à la longueur relativement petite de la cage de manière à l'implanter entre des disques cervicaux.

## Description

La présente invention a trait à une cage intervertébrale destinée à être insérée par voie antérieure à la place d'un disque déficient entre deux corps vertébraux de deux vertèbres cervicales. Une telle cage permet la fusion des corps vertébraux après dissectomie du disque, tout en étant adaptée à la distance intervertébrale entre les deux corps vertébraux.

Le brevet FR-B-2 717 068 divulgue une cage intervertébrale pour vertèbres lombaires comprenant deux branches sensiblement parallèles pour être introduites entre les corps vertébraux, une charnière reliant des extrémités postérieures des branches, et un moyen manoeuvrable depuis des extrémités antérieures des branches pour écarter angulairement les extrémités antérieures des branches depuis une position fermée vers une position ouverte. Les branches sont en butée l'une contre l'autre à la position fermée de manière à introduire par voie antérieure la cage dans un logement ménagé dans un espace intervertébral. Les branches forment un angle de quelques degrés à la position ouverte afin d'appliquer les branches de la cage contre les corps vertébraux délimitant l'espace intervertébral précité.

Selon les réalisations illustrées dans le brevet FR-B-2 717 068, la charnière est une articulation en chape avec un axe de pivotement monté de manière amovible à travers les extrémités postérieures des deux branches. De manière à ne pas trop écarter les branches, cette cage lombaire doit comporter une butée sur les extrémités postérieures des branches.

Le moyen pour écarter angulairement les extrémités antérieures des branches comprend d'une part une vis vissée dans l'extrémité antérieure de l'une des branches et s'étendant entre les branches, et d'autre part, une pièce d'écartement en contact glissant avec des surfaces d'appui en regard sur des faces internes des branches et solidaire en direction axiale avec l'extrémité postérieure de la vis. La pièce d'écartement glisse entre les surfaces d'appui centrales longitudinalement aux branches, situées entre les extrémités antérieures et postérieures des branches, ce qui fragilise les branches.

Ainsi, cette cage comporte cinq pièces à réaliser séparément, et présente des dimensions relativement grandes comparativement à celles d'un disque à remplacer entre deux vertèbres cervicales.

Cette cage est donc d'un coût relativement élevé et n'est pas adaptée au rachis cervical.

La présente invention a pour objectif de fournir une cage intervertébrale ne présentant pas les inconvénients des cages connues, de manière à ce qu'elle contienne un plus petit nombre de pièces et soit adaptée aux dimensions des disques cervicaux, tout en étant implantable par voie antérieure.

Plus particulièrement, l'invention vise à fournir une cage intervertébrale telle que définie dans le préambule de la revendication 1 et ne présentant pas les inconvénients de l'implant intervertébral décrit dans la demande de brevet allemand DE-A-19529605, c'est-à-dire une cage intervertébrale présentant des branches ayant une flexion relativement élevée comparativement à la longueur relativement petite de la cage de manière à implanter la cage entre des disques cervicaux, et non pas des disques lombaires.

A cette fin, une cage intervertébrale pour être insérer entre des vertèbres cervicales, comprend un corps monolithique constitué par deux branches sensiblement parallèles et un moyen de liaison postérieure reliant des extrémités postérieures des branches, et une vis ayant une tige filetée qui s'étend entre les branches et est vissée dans le moyen de liaison postérieure, et une tête à mettre en contact avec des surfaces d'appui en regard dans des extrémités antérieures des branches pour écarter angulairement les extrémités antérieures des branches, est caractérisée en ce que le moyen de liaison comporte un bossage dans lequel la tige de vis est vissée et qui saille dans une double lumière pratiquée dans le corps de cage monolithique entre les branches et le moyen de liaison.

Le bossage, par exemple rectangulaire, offre un plus long taraudage pour la tige de vis ce qui facilite le vissage/dévissage de la vis, tout en renforçant le moyen de liaison et particulièrement une zone centrale du corps monolithique comprise entre des portions postérieures des branches jouant le rôle de charnières "souples". Pour une longueur donnée de la cage, le bossage autorise une plus grande flexion au niveau de "charnières" souples et une épaisseur du moyen de liaison plus petite. La longueur de la cage peut être alors plus réduite.

La cage intervertébrale conforme à l'invention ne comprend ainsi que deux éléments séparables, le corps de cage et la vis, et peut être réduite à des dimensions compatibles avec l'espace intervertébral entre deux vertèbres cervicales.

Le glissement de la tête de vis entre les surfaces d'appui est facilité lorsque la vis comporte un épaulement convexe entre la tête de vis et la tige de vis, à mettre en contact avec les surfaces d'appui qui sont sensiblement tronconiques et convergent en direction du moyen de liaison postérieure lorsque la vis n'écarte pas les branches.

Afin que la cage soit compacte et la vis ne blesse pas le milieu osseux environnant, la tête de vis a un diamètre compris entre les diamètres de petite et grande bases des surfaces d'appui tronconiques lorsque la vis n'écarte pas les branches, et une longueur inférieure à la longueur du corps de cage. La tête de vis est alors sensiblement en retrait de chants des extrémités antérieures des branches lorsque la tête de vis est en contact avec les surfaces d'appui.

Au repos, sans sollicitation des branches par la tête de vis, de préférence la hauteur de chaque branche est inférieure à la demi-hauteur du corps de cage, et les branches sont séparées entièrement par une fente longitudinale s'étendant depuis le moyen de liaison postérieure. Cette fente permet de rapprocher, si nécessaire, les branches lors de l'implantation de la cage dans un logement osseux, et surtout de réaliser des charnières postérieures "souples" monolithiques avec le reste du corps pour écarter les branches à l'angle de lordose souhaité.

Selon une réalisation préférée, la fente s'étend entre les branches depuis les extrémités antérieures de branches jusqu'au moyen de liaison, et comporte une portion antérieure incluant lesdites surfaces d'appui pour la tête de vis, une portion intermédiaire plus étroite et une portion postérieure plus large devant le moyen de liaison. La tête de vis écarte ainsi les branches à l'encontre de leur élasticité propre, c'est-à-dire à l'encontre d'un effort d'autant plus important que l'écartement des branches est grand.

Les branches peuvent comporter alors chacune une portion centrale antérieure large dans laquelle est ménagée l'une des surfaces d'appui pour la tête de vis, deux portions latérales plus étroites, et deux portions de charnière postérieures liées à des extrémités latérales du moyen de liaison et séparées par la portion de fente postérieure plus large.

Des faces externes des portions centrales antérieures et les portions latérales des branches comprennent de préférence des dentures d'ancrage, par exemple transversales, afin de favoriser ultérieurement un contact intime entre le corps de cage et les corps vertébraux.

Les branches peuvent être des chants ayant un contour en forme de tenon de préhension, par exemple en queue d'aronde, de manière à saisir la cage par des mâchoires à profil complémentaire du tenon, d'un outil de préhension, pour introduire la cage dans un logement ménagé entre deux corps vertébraux cervicaux.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 est une vue longitudinale de côté dans un plan antéro-postérieur d'une cage intervertébrale selon l'invention, en position de repos ;
- la figure 2 est une vue de dessus de la face supérieure du corps de la cage selon l'invention ;
- la figure 3 est une vue en coupe axiale prise le long de la ligne III-III de la figure 1, montrant notamment la face inférieure d'une branche supérieure du corps de cage ;
- la figure 4 est une vue frontale distale antérieure de la cage en position de repos ;
- la figure 5 est une vue frontale proximale postérieure de la cage en position de repos ;
- la figure 6 et une vue en perspective postéro-antérieure de dessus éclatée de la cage en position de repos ;
- la figure 7 et une vue en perspective antéro-postérieure de dessous du corps de cage en position de repos ; et
- la figure 8 est une vue en coupe antéro-postérieure prise le long de la ligne médiane VIII-VIII de la figure 5, lorsque la cage est en position de travail, les branches de la cage étant écartées par une tête de vis vissée dans le corps de cage.

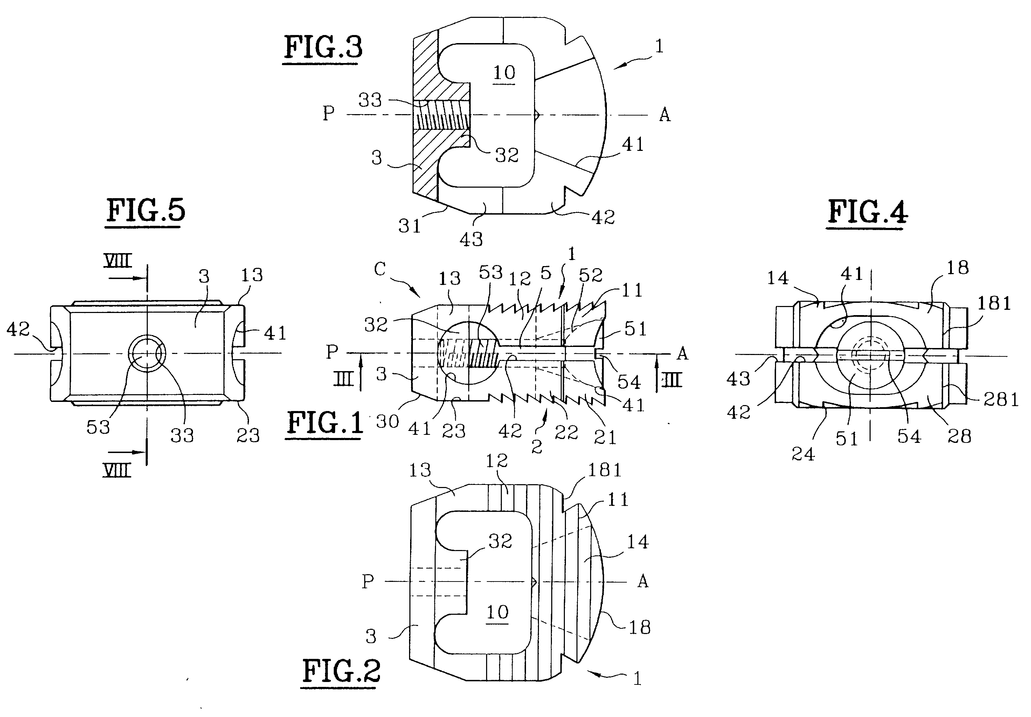

Une cage prothétique intervertébrale selon l'invention est sensiblement parallélépipédique et symétrique par rapport à un axe longitudinal antéro-postérieur A-P. La cage est décrite ci-après en référence aux figures 1 à 7 pour une position de repos, dite également "position fermée".

La cage ne comporte que deux éléments séparables : un corps de cage monolithique C et une vis 5.

Le corps C à un profil postéro-antérieur sensiblement en forme de C. Il est composé essentiellement de trois portions 1, 2 et 3 venues d'usinage et qui sont plus visibles, comme montré à la figure 1, dans un plan antéro-postérieur contenant l'axe A-P, après implantation de la cage. Les trois portions sont deux branches sensiblement rectangulaires 1 et 2 et une âme de liaison 3.

Les branches ont chacune une hauteur inférieure à la moitié de la hauteur hors tout de la cage et s'étendent symétriquement par rapport à un plan antéro-postérieur qui est sensiblement perpendiculaire au rachis cervical lorsque la cage est implantée. Au centre des deux branches 1 et 2 sont ménagées deux grandes lumières sensiblement rectangulaires 10 et 20 traversant les branches de bas en haut de manière à alléger la cage et pouvoir y introduire des greffons osseux.

L'âme de liaison 3 est rectangulaire et s'étend dans un plan frontal entre des extrémités postérieures des deux branches 1 et 2 et a des chants supérieur et inférieur 30 et des chants latéraux 31 chanfreinés et convergeant vers la direction postérieure. L'âme de liaison 3 présente un bossage rectangulaire 32 s'étendant perpendiculairement à l'âme, c'est-à-dire de bas en haut, avec une hauteur sensiblement inférieure à celle du corps de cage au repos, et saillant sur la médiatrice des côtés postérieurs des lumières 10 et 20. Le bossage 32 et l'âme 3 sont percés d'un trou cylindrique taraudé 33 suivant l'axe antéro-postérieur de symétrie A-P.

Dans le sens antéro-postérieur, chaque branche 1, 2 comporte une portion centrale antérieure large 11, 21, deux portions latérales parallèles plus étroites 12, 22 et deux portions de charnière postérieures parallèles 13, 23 liées à des extrémités latérales de l'âme de liaison 3. Les faces externes de chaque branche 1, 2 c'est-à-dire de chaque ensemble de portions 11-12-13, 21-22-23 sont sensiblement coplanaires et parallèles à l'axe A-P. Seulement les faces externes des portions centrales 11 et 21 et des portions latérales 12 et 22 comprennent des dentures transversales en dents de scie 14 et 24 contribuant à un ancrage de la cage dans des corps vertébraux après implantation de la cage et croissance osseuse. En variante, l'une des deux dentures 14 et 24 est supprimée, ou bien au moins l'une des deux dentures est remplacée par des picots de préférence coniques pointus.

Comme cela apparaît dans les figures 1, 6 et 7, les branches 1 et 2 sont séparées par une fente qui s'étend dans un plan antéro-postérieur contenant l'axe A-P et positionné sensiblement perpendiculairement au rachis cervical après implantation de la cage. La fente est constituée d'une portion tronconique lisse antérieure 41 entre les portions centrales de branche 11 et 21, une portion intermédiaire progressivement plus étroite 42, avec des parois planes parallèles sensiblement à angle droit, entre les portions latérales de branche 12 et 22, et une portion postérieure, lisse et sensiblement cylindrique 43 entre les portions de charnière postérieures 13 et 23 des branches.

La portion tronconique 41 de la fente en partie antérieure du corps de cage converge vers le trou central 33 dans le bossage postérieur 32 et a une petite base postérieure ayant un diamètre sensiblement égal au diamètre à fond de filet du trou taraudé 33. La vis 5 a une tête 51 ayant un contour sensiblement en goutte de suif et plus particulièrement un épaulement 52, où prend naissance la tige 53 de la vis qui est convexe, sensiblement en calotte sphérique. La tête de vis 51 a son plus grand diamètre nettement plus grand que celui de la petite base de la portion de fente tronconique 41, et sensiblement plus petit que la grande base de la portion de fente 41. La hauteur de la tête de vis 51 est sensiblement de l'ordre de la demi-longueur de la portion de fente 41.

La tige de vis 53 a une extrémité postérieure filetée pour être vissée dans le trou taraudé 33.

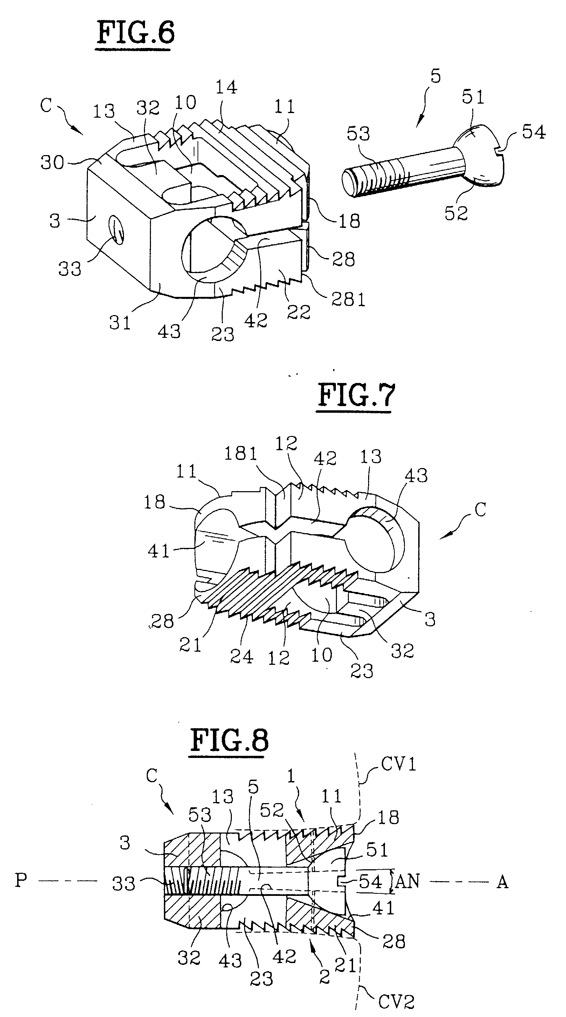

La longueur de la vis 5 est inférieure à la longueur du corps de cage C, mais peut être également inférieure à la longueur de la fente 41-42-43 entre les branches 1 et 2 afin que la vis 5 soit toujours contenue dans le corps de cage C, quelle que soit la position de la cage et donc quel que soit l'écartement des branches 1 et 2 imposé par la tête de vis 51. Lorsque la vis est vissée dans le trou taraudé 33 sans écarter les branches 1 et 2, comme montré à la figure 1, la face fendue de la tête de tige 51 affleure des chants antérieurs 18 et 28 des branches ou est en retrait de ces chants dans la portion tronconique 41, et l'extrémité postérieure de la tige de vis 53 est relativement éloignée de l'extrémité postérieure du trou taraudé 33. De même, lorsque les branches 1 et 2 sont au travail en réponse à une pression exercée par l'épaulement de vis 52 sur la portion tronconique d'appui 41, la tête de vis 51 est plus nettement en retrait des chants antérieurs 18 et 28 des branches 1 et 2, et l'extrémité taraudée de la vis 5 est encore à l'intérieur du trou 33, comme montré à la figure 8.

Le chant antérieur 18, 28 de chacune des branches sensiblement au niveau des portions centrales 11 et 21 a un profil en largeur arrondi, visible aux figures 2 et 3, qui est terminé sensiblement entre la portion centrale 11, 21 et les deux portions latérales 12, 22 de la branche par deux encoches 181, 281 afin que les portions centrales antérieures 11 et 21 constituent un tenon de préhension en queue d'aronde. Le tenon en queue d'aronde 181-281 constitue un moyen de préhension démontable pour un outil, analogue à l'outil porte-cage décrit dans le brevet FR-B-2 717 068. En particulier, l'outil porte-cage comporte à une extrémité proximale d'un long corps tubulaire deux mâchoires longitudinales en regard qui sont maintenues par ressort contre un trou évasé du corps tubulaire et dont les extrémités proximales ont des rainures en queue d'aronde en vis-à-vis. En vissant une tête de manoeuvre taraudée sur l'extrémité distale filetée d'une tige traversant le corps tubulaire, la tige coulisse longitudinalement vers l'extrémité postérieure du corps afin de rapprocher les mâchoires et ainsi prendre le tenon 181-281 à l'extrémité antérieure de la cage.

La dissectomie par voie antérieure pour ménager un logement dans un disque déficient entre deux corps vertébraux cervicaux CV1 et CV2 est pratiquée par râpage de l'espace intervertébral entre les corps vertébraux au moyen d'outils appropriés. Par exemple, une râpe dont la tête a une forme générale pyramidale tronquée à section rectangulaire convergeant en direction postérieure et sensiblement analogue au contour du corps de cage intervertébral C selon l'invention, a un corps guidé dans un cylindre creux, comme décrit dans le brevet FR-B-2 715 293. Le rapâge aboutit à la formation d'un logement de dimensions analogues à celles de la cage.

Puis l'extrémité antérieure en queue d'aronde 181-281 du corps de cage C portant la vis 5 complètement incluse dans celui-ci est saisie par l'outil porte-cage précité, afin que la cage soit implantée par voie antérieure dans le logement formé entre les corps vertébraux CV1 et CV2 en maintenant et positionnant la cage grâce au porte-cage. Les chants chanfreinés postérieurs 30 et 31 de l'âme 3 du corps de cage facilitent la pénétration du corps C dans le logement. Si nécessaire, les branches 1 et 2 peuvent se rapprocher en fléchissant au niveau des portions de charnière 13 et 23 et ainsi réduisant notamment la largeur de la portion de fente étroite 42, ce qui facilite également la pénétration du corps C dans le logement.

Lorsque la cage est complètement située dans le logement intervertébral à l'emplacement souhaité, le cas échéant après avoir frappé sur la tête de l'outil porte-cage, l'outil porte-cage est retiré en dévissant la tête de celui-ci, ce qui écarte les mâchoires à l'extrémité postérieure proximale de l'outil porte-cage de manière à dissocier l'outil porte-cage et le corps de cage.

L'extrémité d'un tournevis est ensuite introduite dans une fente 54 de la tête de vis 51 pour visser la vis dans le trou taraudé 33 et ainsi déplacer la vis 5 axialement dans la fente entre les branches 1 et 2 en direction postérieure. Au début du vissage, l'épaulement de tête de vis 52 vient buter contre la portion tronconique de fente 41. Puis le vissage contraint l'épaulement 52 à exercer des poussées sur les deux surfaces d'appui de branche constituant la portion de fente tronconique 41 de manière à écarter les branches 1 et 2 d'un angle AN de quelques degrés, comme montré à la figure 8. L'angle AN est adapté à l'angle de lordose plus ou moins accentué en fonction de la physiologie du patient. L'écartement des branches est effectué grâce aux paires de portions de charnière 13 et 23 qui jouent le rôle de charnières opposées par rapport au plan de symétrie antéro-postérieur de la cage sensiblement perpendiculaire au rachis cervical, lesquelles portions de charnière sont monolithiques avec le corps de cage. Grâce à cet écartement, les faces externes des branches supérieure et inférieure 1 et 2 de la cage sont mises en contact intime avec les corps vertébraux CV1 et CV2 respectivement. Les dentures 14 et 24 ancrent la cage entre les deux corps vertébraux. Plus l'écartement requis est grand, plus l'effort demandé lors du vissage de la vis 5 à l'encontre de l'élasticité propre des portions de charnière 13 et 23 est élevé, ce qui ne nécessite aucune butée de fin de course d'écartement des branches.

L'écartement angulaire AN des deux branches 1 et 2 de la cage C adapté à l'inclinaison relative des deux corps vertébraux CV1 et CV2 dans un plan antéro-postérieur correspond sensiblement à une augmentation de l'épaisseur de la cage à son extrémité antérieure de quelques dixièmes de mm à 2 mm environ.

Les lumières 10 et 20 favorisent une croissance osseuse intervertébrale ultérieure de sorte que la cage soit complètement noyée dans le milieu osseux. Des greffons osseux peuvent remplir préalablement les lumières.

La cage intersomatique est de préférence en titane et est réalisable en plusieurs tailles de manière à être adaptée à l'anatomie du patient. Le matériel ancillaire dont les dimensions sont fonction de celles de la cage est en conséquence adapté aux tailles de la cage.

A titre d'exemple, la cage a une hauteur (épaisseur) hors tout de 7 mm, une longueur suivant l'axe antéro-postérieur A-P de 12,5 mm, et une largeur hors tout comprise entre 12 et 15 mm suivant la taille de la cage. La hauteur de la portion intermédiaire de fente 42 entre les porterons latérales de branche 12 et 22 est de 1 mm au repos. L'angle au sommet de la portion de fente tronconique 41 est de 36°. Le diamètre de la portion cylindrique de fente 43 est de 4,4 mm. L'épaisseur de l'âme 3 suivant l'axe A-P est de 1,5 mm avec un bossage 32 de hauteur de 2,3 mm par rapport à une longueur de lumière 10 et 20 de 6,5 mm.

## Revendications

1. Cage intervertébrale comprenant un corps monolithique (C) constitué par deux branches sensiblement parallèles (1, 2) et un moyen de liaison postérieure (3) reliant des extrémités postérieures (13, 23) des branches, et une vis (5) ayant une tige filetée (53) qui s'étend entre les branches (1, 2) et est vissée dans le moyen de liaison postérieure (3), et une tête (51) à mettre en contact avec des surfaces d'appui en regard (41) dans des extrémités antérieures (11, 21) des branches (1, 2) pour écarter angulairement les extrémités antérieures des branches, caractérisée en ce que le moyen de liaison (3) comporte un bossage (32) dans lequel la tige de vis (52) est vissée et qui saille dans une double lumière (10, 20) pratiquée dans le corps de cage monolithique (C) entre les branches (1, 2) et le moyen de liaison.

2. Cage intervertébrale conforme à la revendication 1, dans laquelle la vis (5) comporte un épaulement convexe (52) entre la tête de vis (51) et la tige de vis (53), à mettre en contact avec les surfaces d'appui (41) qui sont sensiblement tronconiques et convergent en direction du moyen de liaison postérieure lorsque la vis n'écarte pas les branches.

3. Cage intervertébrale conforme à la revendication 2, dans laquelle la tête de vis (51) a un diamètre compris entre les diamètres de petite et grande bases des surfaces d'appui tronconiques (41) lorsque la vis n'écarte pas les branches (1, 2).

4. Cage intervertébrale conforme à l'une quelconque des revendications 1 à 3, dans laquelle la vis (5) a une longueur inférieure à la longueur du corps de cage (C), et la tête de vis (51) est sensiblement en retrait de chants (18, 28) des extrémités antérieures (11, 21) des branches (1, 2) lorsque la tête de vis (51) est en contact avec les surfaces d'appui (41).

5. Cage intervertébrale conforme à l'une quelconque des revendications 1 à 4, dans laquelle la hauteur de chaque branche (1, 2) est inférieure à la demi-hauteur du corps de cage (C), et les branches sont séparées entièrement par une fente longitudinale (41, 42, 43) s'étendant depuis le moyen de liaison postérieure (3).

6. Cage intervertébrale conforme à l'une quelconque des revendications 1 à 5, comprenant une fente s'étendant entre les branches (1, 2) depuis les extrémités antérieures de branche (11, 21) jusqu'au moyen de liaison (3), et comportant une portion antérieure (41) incluant lesdites surfaces d'appui pour la tête de vis (51), une portion intermédiaire plus étroite (42), et une portion postérieure plus large (43) devant le moyen de liaison (3).

7. Cage intervertébrale conforme à l'une quelconque des revendications 1 à 6, dans laquelle les branches (1, 2) comportent chacune une portion centrale antérieure large (11, 21) dans laquelle est ménagée l'une des surfaces d'appui pour la tête de vis (41), deux portions latérales plus étroites (12, 22), et deux portions de charnière postérieures (13, 23) liées à des extrémités latérales du moyen de liaison (3).

8. Cage intervertébrale conforme à la revendication 7, dans laquelle des faces externes des portions centrales antérieures (11, 21) et des portions latérales (12, 22) des branches (1, 2) comprennent des dentures d'ancrage (14, 24).

9. Cage intervertébrale conforme à l'une quelconque des revendications 1 à 8, dans laquelle les extrémités antérieures (11, 21) des branches (1, 2) ont des chants (18, 181 ; 28, 281) ayant un contour en forme de tenon de préhension.
